(19) Europäisches Patentamt
European Patent Office
Office européen des brevets

(11) EP 1 670 313 B1

(12) **EUROPÄISCHE PATENTSCHRIFT**

(45) Veröffentlichungstag und Bekanntmachung des Hinweises auf die Patenterteilung:
**14.02.2007 Patentblatt 2007/07**

(21) Anmeldenummer: **04787060.5**

(22) Anmeldetag: **30.09.2004**

(51) Int Cl.:
***A01N 43/90*** *(2006.01)* ***C07D 487/04*** *(2006.01)*

(86) Internationale Anmeldenummer:
**PCT/EP2004/010918**

(87) Internationale Veröffentlichungsnummer:
**WO 2005/032256 (14.04.2005 Gazette 2005/15)**

(54) **FUNGIZIDE MISCHUNGEN**

FUNGICIDAL MIXTURES

MÉLANGES FONGICIDES

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**HR LT LV**

(30) Priorität: **01.10.2003 DE 10346136**

(43) Veröffentlichungstag der Anmeldung:
**21.06.2006 Patentblatt 2006/25**

(73) Patentinhaber: **BASF Aktiengesellschaft**
**67056 Ludwigshafen (DE)**

(72) Erfinder:
- **TORMO I BLASCO, Jordi**
  **69514 Laudenbach (DE)**
- **GROTE, Thomas**
  **67157 Wachenheim (DE)**
- **SCHERER, Maria**
  **76829 Godramstein (DE)**
- **STIERL, Reinhard**
  **67251 Freinsheim (DE)**
- **STRATHMANN, Siegfried**
  **67117 Limburgerhof (DE)**
- **SCHÖFL, Ulrich**
  **68782 Brühl (DE)**

(56) Entgegenhaltungen:
**EP-A- 0 988 790**
**WO-A-98/46607**
**WO-A-20/04064519**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft fungizide Mischungen, enthaltend als aktive Komponenten

1) das Triazolopyrimidinderivat der Formel I,

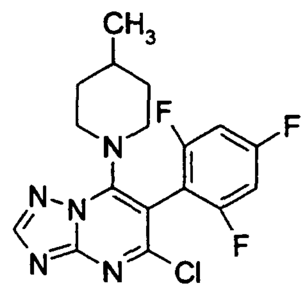

I

und

2) Tebuconazole der Formel II,

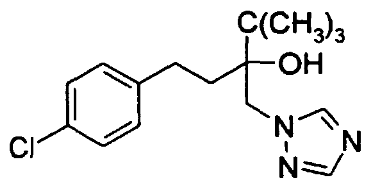

II

in einer synergistisch wirksamen Menge.

Außerdem betrifft die Erfindung ein Verfahren zur Bekämpfung von Schadpilzen aus der Klasse der *Oomyceten* mit Mischungen der Verbindung I mit der Verbindung II und die Verwendung der Verbindung I mit der Verbindung II zur Herstellung derartiger Mischungen sowie Mittel, die diese Mischungen enthalten.

Die Verbindung I, 5-Chlor-7-(4-methyl-piperidin-1-yl)-6-(2,4,6-trifluor-phenyl)-[1,2,4]triazolo[1,5-a]pyrimidin, ihre Herstellung und deren Wirkung gegen Schadpilze ist aus der Literatur bekannt (WO 98/46607).

Die Verbindung II, (R,S)-1-(4-Chlorphenyl)-4,4-dimethyl-3-(1H-1,2,4-triazol-1-ylmethyl)pentan-3-ol, ihre Herstellung und deren Wirkung gegen Schadpilze ist ebenfalls aus der Literatur bekannt (EP-A 40 345; common name: Tebuconazole). Tebuconazole ist seit Langem als Getreidefungizid im Markt etabliert.

Mischungen von Triazolopyrimidinderivaten mit Tebuconazole sind allgemein aus EP-A 988 790 bekannt. Die Verbindung I ist von der allgemeinen Offenbarung dieser Schrift umfasst, ist jedoch nicht explizit erwähnt. Die Kombination der Verbindung I mit Tebuconazole ist daher neu.

Die in EP-A 988 790 beschriebenen synergistischen Mischungen von Triazolopyrimidinen werden als fungizid wirksam gegen verschiedene Krankheiten von Getreide, Obst und Gemüse, insbesondere Mehltau an Weizen und Gerste oder Grauschimmel an Äpfeln beschrieben. Die fungizide Wirkung dieser Mischungen gegen Schadpitze aus der Klasse der *Oomyceten* lässt jedoch zu wünschen übrig.

Das biologische Verhalten von *Oomyceten* weicht deutlich von dem der *Ascomyceten, Deuteromyceten,* und *Basidiomyceten* ab, denn *Oomyceten* sind biologisch eher mit Algen als mit Pilzen verwandt. Daher sind Erkenntnisse zur fungiziden Aktivität von Wirkstoffen gegen "echte Pilze", wie *Ascomyceten, Deuteromyceten,* und *Basidiomyceten* nur sehr eingeschränkt auf *Oomyceten* übertragbar.

*Oomyceten* verursachen wirtschaftlich bedeutsame Schäden an verschiedenen Kulturpflanzen. In vielen Regionen stellen Infektionen durch *Phytophthora infestans* im Kartoffel- und Tomatenanbau die bedeutendsten Pflanzenkrankheiten dar. Im Weinbau werden erhebliche Schäden durch Rebenperonospora verursacht.

Es besteht ein andauemder Bedarf an neuen Oomyceten-Mitteln in der Landwirtschaft, da die Schadpilze gegen die im Markt etablierten Produkte, wie z.B. Metalaxyl und strukturell ähnliche Wirkstoffe, bereits verbreitet Resistenzen entwickelt haben.

Praktische Erfahrungen in der Landwirtschaft haben gezeigt, dass der wiederholte und ausschließliche Einsatz eines Einzelwirkstoffs bei der Bekämpfung von Schadpilzen in vielen Fällen zur schnellen Selektion von solchen Pilzstämmen führt, die gegen den betreffenden Wirkstoff eine natürliche oder adaptierte Resistenz entwickelt haben. Eine wirksame Bekämpfung dieser Pilze mit dem betreffenden Wirkstoff ist dann nicht mehr möglich.

Um die Gefahr der Selektion von resistenten Pilzstämmen zu verringern, werden heutzutage zur Bekämpfung von Schadpilzen bevorzugt Mischungen verschiedener Wirkstoffe eingesetzt. Durch Kombination von Wirkstoffen mit unterschiedlichen Wirkungsmechanismen kann der Bekämpfungserfolg über längere Zeit gesichert werden.

Im Hinblick auf effektives Resistenzmanagement und eine wirkungsvolle Bekämpfung von Schadpilzen aus der Klasse der *Oomyceten* bei möglichst geringen Aufwandmengen lagen der vorliegenden Erfindung Mischungen als Aufgabe zugrunde, die bei möglichst geringer Gesamtmenge an ausgebrachten Wirkstoffen eine ausreichende Wirkung gegen die Schadpilze zeigen.

Demgemäss wurden die eingangs definierten Mischungen gefunden. Es wurde außerdem gefunden, dass sich bei gleichzeitiger gemeinsamer oder getrennter Anwendung der Verbindung I und der Verbindung II oder bei Anwendung der Verbindungen I und der Verbindung II nacheinander *Oomyceten* besser bekämpfen lassen als mit den Einzelverbindungen (synergistische Mischungen).

Die Mischungen der Verbindung I und der Verbindung 11 bzw. die gleichzeitige gemeinsame oder getrennte Verwendung der Verbindung I und der Verbindung II zeichnen sich aus durch eine hervorragende Wirksamkeit gegen pflanzenpathogene Pilze aus der Klasse der *Oomyceten,* insbesondere von *Phytophthora infestans* an Kartoffeln und Tomaten, sowie *Plasmopara viticola* an Reben. Sie können im Pflanzenschutz als Blatt- und Bodenfungizide eingesetzt werden.

Besondere Bedeutung haben sie für die Bekämpfung von *Oomyceten* an verschiedenen Kulturpflanzen wie Gemüsepflanzen (z.B. Gurken, Bohnen und Kürbisgewächse), Kartoffeln, Tomaten, Reben und entsprechende Samen.

Insbesondere eignen sie sich zur Bekämpfung der Kraut- und Knollenfäule an Tomaten und Kartoffeln, die durch *Phytophthora infestans* verursacht wird, sowie des falschen Rebenmehltaus (Rebenperonospora), verursacht durch *Plasmopara viticola.*

Darüber hinaus ist die erfindungsgemäße Kombination der Verbindungen I und II auch zur Bekämpfung anderer Pathogene geeignet, wie z. B. *Septoria*- und *Puccinia*-Arten in Getreide und *Alternaria*- und *Boytritis*-Arten in Gemüse, Obst und Wein.

Bevorzugt setzt man bei der Bereitstellung der Mischungen die reinen Wirkstoffe I und II ein, denen man je nach Bedarf weitere Wirkstoffe gegen Schadpilze oder andere Schädlinge wie Insekten, Spinntiere oder Nematoden, oder auch herbizide oder wachstumsregulierende Wirkstoffe oder Düngemittel beimischen kann.

Als weitere Wirkstoffe im voranstehenden Sinne kommen insbesondere Fungizide ausgewählt aus der folgenden Gruppe in Frage:

- Acylalanine wie Benalaxyl, Metalaxyl, Ofurace, Oxadixyl,
- Aminderivate wie Aldimorph, Dodemorph, Fenpropidin, Guazatine, Iminoctadine, Tridemorph,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Enilconazol, Fenbuconazol, Fluquiconazol, Flusilazol, Flutriafol, Hexaconazol, Imazalil, Ipconazol, Myclobutanil, Penconazol, Propiconazol, Prochloraz, Prothioconazol, Simeconazol, Tetraconazol, Triadimefon, Triadimenol, Triflumizol, Triticonazol,
- Dicarboximide wie Myclozolin, Procymidon,
- Dithiocarbamate wie Ferbam, Nabam, Metam, Propineb, Polycarbamat, Ziram, Zineb,
- Heterocylische Verbindungen wie Anilazin, Boscalid, Carbendazim, Carboxin, Oxycarboxin, Cyazofamid, Dazomet, Famoxadon, Fenamidon, Fuberidazol, Flutolanil, Furametpyr, Isoprothiolan, Mepronil, Nuarimol, Probenazol, Pyroquilon, Silthiofam, Thiabendazol, Thifluzamid, Tiadinil, Tricyclazol, Triforine,
- Nitrophenylderivate wie Binapacryl, Dinocap, Dinobuton, Nitrophthal-isopropyl,
- Phenylpyrrole wie Fenpiclonil oder Fludioxonil,
- Schwefel,
- Sonstige Fungizide wie Acibenzolar-S-methyl, Carpropamid, Chlorothalonil, Cyflufenamid, Cymoxanil, Diclomezin, Diclocymet, Diethofencarb, Edifenphos, Ethaboxam, Fenhexamid, Fentin-Acetat, Fenoxanil, Ferimzone, Fluazinam, Fosetyl, Hexachlorbenzol, Metrafenon, Pencycuron, Propamocarb, Phthalid, Toloclofosmethyl, Quintozene, Zoxamid,
- Strobilurine wie Fluoxastrobin, Metominostrobin, Orysastrobin, Pyraclostrobin oder Trifloxystrobin,
- Sulfensäurederivate wie Captafol,
- Zimtsäureamide und Analoge wie Flumetover.

In einer Ausführungsform der erfindungsgemäßen Mischungen werden den Verbindungen I und II ein weiteres Fungizid III oder zwei Fungizide III und IV beigemischt. Mischungen der Verbindungen I und II mit einer Komponente III sind bevorzugt. Besonders bevorzugt sind Mischungen der Verbindungen I und II.

Die Verbindung I und die Verbindung II können gleichzeitig gemeinsam oder getrennt oder nacheinander

aufgebracht werden, wobei die Reihenfolge bei getrennter Applikation im allgemeinen keine Auswirkung auf den Bekämpfungserfolg hat.

Die Verbindung I und die Verbindung II werden üblicherweise in einem Gewichtsverhältnis von 100:1 bis 1: 100, vorzugsweise 10:1 bis 1:50, insbesondere 5:1 bis 1:10 angewandt.

Die Komponenten III und ggf. IV werden gewünschtenfalls im Verhältnis von 20:1 bis 1:20 zu der Verbindung I zugemischt.

Die Aufwandmengen der erfindungsgemäßen Mischungen liegen je nach Art der Verbindung und des gewünschten Effekts bei 5 g/ha bis 2000 g/ha, vorzugsweise 50 bis 1500 g/ha, insbesondere 50 bis 750 g/ha.

Die Aufwandmengen für die Verbindung I liegen entsprechend in der Regel bei 1 bis 1000 g/ha, vorzugsweise 10 bis 750 g/ha, insbesondere 20 bis 500 g/ha.

Die Aufwandmengen für die Verbindung II liegen entsprechend in der Regel bei 5 bis 2000 g/ha, vorzugsweise 10 bis 1000 g/ha, insbesondere 50 bis 750 g/ha.

Bei der Saatgutbehandlung werden im allgemeinen Aufwandmengen an Mischung von 1 bis 1000 g/100kg Saatgut, vorzugsweise 1 bis 750 g/100 kg, insbesondere 5 bis 500 g/100 kg verwendet.

Bei der Bekämpfung für Pflanzen pathogener Schadpilze erfolgt die getrennte oder gemeinsame Applikation der Verbindung I und der Verbindung II oder der Mischungen aus der Verbindung I und der Verbindung II durch Besprühen oder Bestäuben der Samen, der Pflanzen oder der Böden vor oder nach der Aussaat der Pflanzen oder vor oder nach dem Auflaufen der Pflanzen.

Die erfindungsgemäßen Mischungen, bzw. die Verbindungen I und II können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z. B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:

- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubmittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im allgemeinen zwischen 0,01 und 95 Gew.%, vorzugsweise zwischen 0,1 und 90 Gew.% der Wirkstoffe. Die Wirkstoffe werden dabei in einer Reinheit von 90% bis 100%, vorzugsweise 95% bis 100% (nach NMR-Spektrum) eingesetzt.

Beispiele für Formulierungen sind: 1. Produkte zur Verdünnung in Wasser

A) Wasserlösliche Konzentrate (SL)
10 Gew.-Teile der Wirkstoffe werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.

B) Dispergierbare Konzentrate (DC)
20 Gew.-Teile der Wirkstoffe werden in Cyclohexanon unter Zusatz eines Dispergiermittels z.B. Polyvinylpyrrolidon gelöst. Bei Verdünnung in Wasser ergibt sich eine Dispersion.

C) Emulgierbare Konzentrate (EC)
15 Gew.-Teile der Wirkstoffe werden in Xylol unter Zusatz von Ca-Dodecylbenzolsulfonat und Ricinusölethoxylat (jeweils 5 %) gelöst. Bei der Verdünnung in Wasser ergibt sich eine Emulsion.

D) Emulsionen (EW, EO)
40 Gew.-Teile der Wirkstoffe werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.

E) Suspensionen (SC, OD)
20 Gew.-Teile der Wirkstoffe werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.

F) Wasserdispergierbare und wasserlösliche Granulate (WG, SG)
50 Gew.-Teile der Wirkstoffe werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.

G) Wasserdispergierbare und wasserlösliche Pulver (WP, SP)
75 Gew.-Teile der Wirkstoffe werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.
2. Produkte für die Direktapplikation

H) Stäube (DP)
5 Gew.Teile der Wirkstoffe werden fein gemahlen und mit 95 % feinteiligem Kaolin innig vermischt. Man erhält dadurch ein Stäubmittel.

I) Granulate (GR, FG, GG, MG)
0.5 Gew-Teile der Wirkstoffe werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.

J) ULV- Lösungen (UL)
10 Gew.-Teile der Wirkstoffe werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubmitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Verbindungen I und II, bzw. die Mischungen oder die entsprechenden Formulierungen werden angewendet, indem man die Schadpilze, die von ihnen freizuhaltenden Pflanzen, Samen, Böden, Flächen, Materialien oder Räume mit einer fungizid wirksamen Menge der Mischung, bzw. der Verbindungen I und II bei getrennter Ausbringung, behandelt. Die Anwendung kann vor oder nach dem Befall durch die Schadpilze erfolgen.

Die fungizide Wirkung der Verbindung und der Mischungen lässt sich durch folgende Versuche zeigen:

Die Wirkstoffe wurden getrennt oder gemeinsam als eine Stammlösung aufbereitet mit 0,25 Gew.% Wirkstoff in Aceton oder DMSO. Dieser Lösung wurde 1 Gew.% Emulgator Uniperol® EL (Netzmittel mit Emulgier- und Dispergierwirkung auf der Basis ethoxylierter Alkylphenole) zugesetzt und entsprechend der gewünschten Konzentration mit Wasser verdünnt.

Anwendungsbeispiel 1-Aktivität gegen die Krautfäule an Tomaten verursacht durch *Phytophthora infestans* bei protektiver Behandlung

Blätter von getopften Tomatenpflanzen wurden mit einer wässriger Suspension in der unten angegebenen Wirkstoffkonzentration bis zur Tropfnässe besprüht. Am folgenden Tag wurden die Blätter mit einer wässrigen Sporangienaufschwemmung von *Phytophthora infestans* infiziert. Anschließend wurden die Pflanzen in einer wasserdampfgesättigten Kammer bei Temperaturen zwischen 18 und 20°C aufgestellt. Nach 6 Tagen hatte sich die Krautfäule auf den unbehandelten, jedoch infizierten Kontrollpflanzen so stark entwickelt, dass der Befall visuell in % ermittelt werden konnte.

Die visuell ermittelten Werte für den Prozentanteil befallener Blattflächen wurden in Wirkungsgrade als % der unbehandelten Kontrolle umgerechnet:

Der Wirkungsgrad (W) wird nach der Formel von Abbot wie folgt berechnet:

$$W = (1 - \alpha/\beta) \cdot 100$$

$\alpha$ entspricht dem Pilzbefall der behandelten Pflanzen in % und

$\beta$ entspricht dem Pilzbefall der unbehandelten (Kontroll-) Pflanzen in %

Bei einem Wirkungsgrad von 0 entspricht der Befall der behandelten Pflanzen demjenigen der unbehandelten Kontrollpflanzen; bei einem Wirkungsgrad von 100 weisen die behandelten Pflanzen keinen Befall auf.

Die zu erwartenden Wirkungsgrade für Wirkstoffkombinationen wurden nach der Colby-Formel (Colby, S. R. (Calculating synergistic and antagonistic responses of herbicide Combinations", Weeds, <u>15,</u> S. 20 - 22, 1967) ermittelt und mit den beobachteten Wirkungsgraden verglichen.

Colby Formel:

$$E = x + y - x \cdot y/100$$

E zu erwartender Wirkungsgrad, ausgedrückt in % der unbehandelten Kontrolle, beim Einsatz der Mischung aus den Wirkstoffen A und B in den Konzentrationen a und b

x der Wirkungsgrad, ausgedrückt in % der unbehandelten Kontrolle, beim Einsatz des Wirkstoffs A in der Konzentration a

y der Wirkungsgrad, ausgedrückt in % der unbehandelten Kontrolle, beim Einsatz des Wirkstoffs B in der Konzentration b

Als Vergleichsverbindungen wurden die von den in EP-A 988 790 beschriebenen Tebuconazole-Mischungen bekannten Verbindungen A und B verwendet:

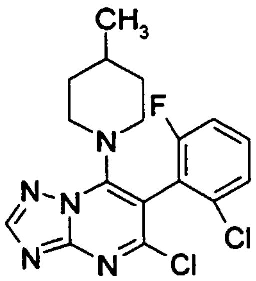
A

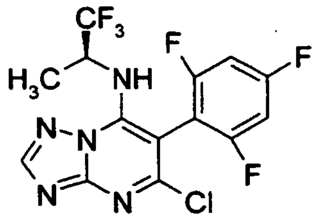
B

Tabelle A - Einzelwirkstoffe

| Beispiel | Wirkstoff | Wirkstoffkonzentration in der Spritzbrühe [ppm] | Wirkungsgrad in % der unbehandelten Kontrolle |
|---|---|---|---|
| 1 | Kontrolle (unbehandelt) | - | (88% Befall) |
| 2 | I | 63 | 9 |
| 3 | II (Tebuconazole) | 16<br>250 | 0 |
| 4 | Vergleich A | 63 | 0 |
| 5 | Vergleich B | 63 | 9 |

Tabelle B - erfindungsgemäße Mischungen

| Beispiel | Wirkstoffmischung Konzentration Mischungsverhältnis | beobachteter Wirkungsgrad | berechneter Wirkungsgrad*) |
|---|---|---|---|
| 6 | I+II<br>63 + 16 ppm<br>4:1 | 43 | 9 |
| 7 | I+II<br>63 + 250 pm<br>1:4 | 55 | 9 |
| *) berechneter Wirkungsgrad nach der Colby-Formel | | | |

Tabelle C - Vergleichsversuche - Mischungen bekannt aus EP-A 988 780

| Beispiel | Wirkstoffmischung Konzentration Mischungsverhältnis | beobachteter Wirkungsgrad | berechneter Wirkungsgrad*) |
|---|---|---|---|
| 8 | A+II<br>63 + 16 ppm<br>4:1 | 0 | 0 |
| 9 | A + II<br>63 + 250 pm<br>1:4 | 0 | 0 |

(fortgesetzt)

| Beispiel | Wirkstoffmischung Konzentration Mischungsverhältnis | beobachteter Wirkungsgrad | berechneter Wirkungsgrad*) |
|---|---|---|---|
| 10 | B+II<br>63 + 16 ppm<br>4:1 | 9 | 9 |
| 11 | B+II<br>63 + 250 pm<br>1:4 | 21 | 9 |
| *) berechneter Wirkungsgrad nach der Colby-Formel | | | |

Aus den Ergebnissen der Versuche geht hervor, dass die erfindungsgemäßen Mischungen durch einen starken Synergismus gegen die Krautfäule erheblich besser wirksam sind, als die aus EP-A 988 780 bekannten Tebuconazole-Mischungen

Anwendungsbeispiel 2 - Wirksamkeit gegen Rebenperonospora verursacht durch *Plasmopara viticola*

Blätter von Topfreben der Sorte "Riesling" wurden mit wässriger Suspension in der unten angegebenen Wirkstoffkonzentration bis zur Tropfnässe besprüht. Am folgenden Tag wurden die Unterseiten der Blätter mit einer wässrigen Zoosporenaufschwemmung von *Plasmopara viticola* inokuliert. Danach wurden die Reben zunächst für 48 Stunden in einer wasserdampfgesättigten Kammer bei 24°C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

Die Auswertung erfogte analog Beispiel 1.

Tabelle D - Einzelwirkstoffe

| Beispiel | Wirkstoff | Wirkstoffkonzentration in der Spritzbrühe [ppm] | Wirkungsgrad in % der unbehandelten Kontrolle |
|---|---|---|---|
| 12 | - | Kontrolle (unbehandelt) | (84 % Befall) |
| 13 | I | 4<br>1 | 52<br>4 |
| 14 | II (Tebuconazole) | 4<br>1 | 28<br>0 |

Tabelle E - erfindungsgemäße Mischungen

| Beispiel | Wirkstoffmischung Konzentration Mischungsverhältnis | beobachteter Wirkungsgrad | berechneter Wirkungsgrad*) |
|---|---|---|---|
| 15 | I+II<br>4+1 ppm<br>4:1 | 76 | 52 |
| 16 | I+II<br>1+4 ppm<br>1:4 | 76 | 31 |
| *) berechneter Wirkungsgrad nach der Colby-Formel | | | |

Aus den Ergebnissen der Versuche geht hervor, dass der beobachtete Wirkungsgrad der erfindungsgemäßen Mischungen in allen Mischungsverhältnissen deutlich höher ist, als nach der Colby-Formel vorausberechnet.

## Patentansprüche

1. Fungizide Mischungen, enthaltend als aktive Komponenten

1) das Triazolopyrimidinderivat der Formel I,

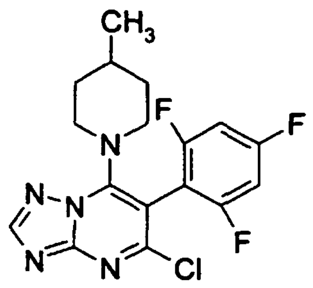

I

und

2) Tebuconazole der Formel II,

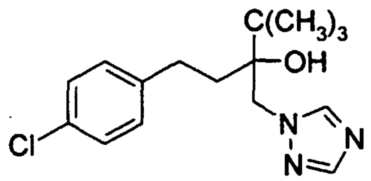

II

in einer synergistisch wirksamen Menge.

2. Fungizide Mischungen, enthaltend die Verbindung der Formel I und die Verbindung der Formel II in einem Gewichtsverhältnis von 100:1 bis 1:100.

3. Fungizides Mittel, enthaltend einen flüssigen oder festen Trägerstoff und eine Mischung gemäß Anspruch 1 oder 2.

4. Verfahren zur Bekämpfung von Schadpilzen aus der Klasse der *Oomyceten,* **dadurch gekennzeichnet, dass** man die Pilze, deren Lebensraum oder die vor Pilzbefall zu schützenden Pflanzen, den Boden oder Saatgüter mit einer wirksamen Menge der Verbindung I und der Verbindung II gemäß Anspruch 1 behandelt.

5. Verfahren nach Anspruch 4, **dadurch gekennzeichnet, dass** man die Verbindungen I und II gemäß Anspruch 1 gleichzeitig, und zwar gemeinsam oder getrennt, oder nacheinander ausbringt.

6. Verfahren nach Anspruch 4, **dadurch gekennzeichnet, dass** man die Mischung gemäß Ansprüchen 1 oder 2 auf die vor Pilzbefall zu schützenden Pflanzen oder den Boden in einer Menge von 5 g/ha bis 2000 g/ha aufwendet.

7. Verfahren nach Ansprüchen 4 und 5, **dadurch gekennzeichnet, dass** man die Mischung gemäß Ansprüchen 1 oder 2 in einer Menge von 1 bis 1000 g/100 kg Saatgut anwendet.

8. Verfahren nach Ansprüchen 4 bis 7, **dadurch gekennzeichnet, dass** der Schadpilz *Phytophthora infestans* bekämpft wird.

9. Saatgut, enthaltend die Mischung gemäß Ansprüchen 1 oder 2 in einer Menge von 1 bis 1000 g/100 kg.

10. Verwendung der Verbindung I und der Verbindung II gemäß Anspruch 1 zur Herstellung eines zur Bekämpfung von *Oomyceten* geeigneten Mittels.

## Claims

1. A fungicidal mixture comprising, as active components,

1) the triazolopyrimidine derivative of the formula I

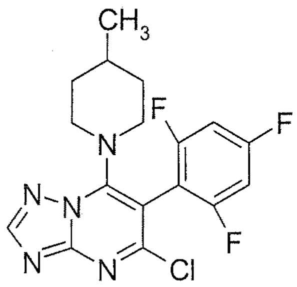

I

and
2) tebuconazole of the formula II,

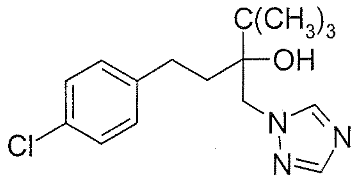

II

in a synergistically effective amount.

2. A fungicidal mixture comprising the compound of the formula I and the compound of the formula II in a weight ratio of from 100:1 to 1:100.

3. A fungicidal composition comprising a liquid or solid carrier and a mixture according to claim 1 or 2.

4. A method for controlling harmful fungi from the class of the *Oomycetes,* which comprises treating the fungi, their habitat or the seed, the soil or the plants to be protected against fungal attack with an effective amount of the compound I and the compound II according to claim 1.

5. The method according to claim 4, wherein the compounds I and II according to claim 1 are applied simultaneously, that is jointly or separately, or in succession.

6. The method according to claim 4, wherein the mixture according to claim 1 or 2 is applied to the soil or the plants to be protected against fungal attack in an amount of from 5 g/ha to 2 000 g/ha.

7. The method according to claim 4 or 5, wherein the mixture according to claim 1 or 2 is applied in an amount of from 1 to 1000 g/100 kg of seed.

8. The method according to any of claims 4 to 7, wherein the harmful fungus *Phytophthora infestans* is controlled.

9. Seed comprising the mixture according to claim 1 or 2 in an amount of from 1 to 1000 g/100 kg.

10. The use of the compound I and the compound II according to claim 1 for preparing a composition suitable for controlling *Oomycetes.*

## Revendications

**1.** Mélanges fongicides contenant, comme composants actifs,

1) le dérivé de triazolopyrimidine de la formule I :

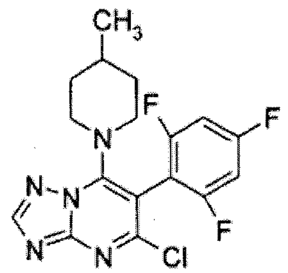

I

et
2) du tébuconazole de la formule II :

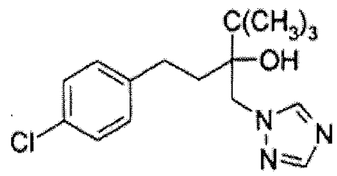

II

en une quantité efficace du point de vue synergique.

**2.** Mélanges fongicides, contenant le composé de la formule I et le composé de la formule II dans un rapport pondéral de 100/1 à 1/100.

**3.** Produit fongicide, contenant un support liquide ou solide et un mélange suivant la revendication 1 ou 2.

**4.** Procédé pour lutter contre les champignons nuisibles de la classe des *Oomycètes,* **caractérisé en ce qu'**on traite les champignons, leur environnement ou les plantes, sols ou semences à protéger contre une infestation par ces champignons par une quantité efficace du composé I et du composé II suivant la revendication 1.

**5.** Procédé suivant la revendication 4, **caractérisé en ce qu'**on épand les composés I et II suivant la revendication 1 soit simultanément, et cela conjointement ou séparément, soit successivement.

**6.** Procédé suivant la revendication 4, **caractérisé en ce qu'**on applique le mélange suivant les revendications 1 ou 2 sur les plantes ou sols à protéger d'une infestation par les champignons en une quantité de 5 g/ha à 2000 g/ha.

**7.** Procédé suivant les revendications 4 et 5, **caractérisé en ce qu'**on applique le mélange suivant les revendications 1 ou 2 en une quantité de 1 à 1000 g/100 kg de semences.

**8.** Procédé suivant les revendications 4 à 7, **caractérisé en ce qu'**on lutte contre le champignon nuisible *Phytophthora infestans.*

**9.** Semences, contenant le mélange suivant les revendications 1 ou 2, en une quantité de 1 à 1000 g/100 kg.

**10.** Utilisation du composé I et du composé II suivant la revendication 1, pour la préparation d'un produit approprié pour la lutte contre les *Oomycètes.*